# FASCICULE DE BREVET EUROPEEN

(11) **EP 3 222 989 B1**
(45) Date de publication et mention de la délivrance du brevet: **22.08.2018**
(21) Numéro de dépôt: 17161753.3
(22) Date de dépôt: 20.03.2017
(51) Int. Cl.: G01N 1/28, C12N 1/06

(54) **DISPOSITIF D'ANALYSE D'UN ÉCHANTILLON BIOLOGIQUE**
ANALYSEVORRICHTUNG FÜR EINE BIOLOGISCHE PROBE
DEVICE FOR ANALYSING A BIOLOGICAL SAMPLE

(30) Priorité: 21.03.2016 FR 1652378
(43) Date de publication de la demande: 27.09.2017
(73) Titulaire: COMMISSARIAT À L'ÉNERGIE ATOMIQUE ET AUX ÉNERGIES ALTERNATIVES, 75015 Paris (FR)
(72) Inventeur: GOSSELIN, David, 38000 GRENOBLE (FR); BERTHIER, Jean, 38240 MEYLAN (FR); BOURDAT, Anne-Gaëlle, 38260 NANTOIN (FR); VENTOSA, Jérome, 38000 GRENOBLE (FR)
(74) Mandataire: GIE Innovation Competence Group

(56) Documents cités:
- WO-A1-99/28742
- WO-A1-2015/181743
- US-A1- 2003 066 915

## Description

### Domaine technique de l'invention

La présente invention se rapporte à un dispositif d'analyse d'un échantillon biologique comportant des espèces biologiques. Ce dispositif d'analyse permet notamment de réaliser une concentration et purification des espèces biologiques présentes dans l'échantillon, une lyse de ces espèces biologiques en vue d'en extraire le matériel biologique à analyser, puis une séparation de ce matériel biologique et une réaction d'amplification en vue d'une détection d'un pathogène dans l'échantillon.

### Etat de la technique

La détection de pathogènes dans un échantillon biologique est souvent réalisée en employant un matériel lourd et peu adapté, notamment pour une analyse rapide sur le terrain. La détection nécessite notamment une étape de lyse des espèces biologiques contenues dans l'échantillon pour broyer lesdites espèces, après des étapes de concentration et de purification.

Des dispositifs qui permettent de mettre en oeuvre les étapes de concentration, de purification et lyse mécanique sont connus de l'état de la technique. La demande de brevet WO2015/181743A1 décrit notamment un tel dispositif. Dans celui-ci, la lyse mécanique est réalisée par cisaillement entre deux parois, l'une des deux parois ayant une surface d'appui rugueuse. Un tel dispositif permet essentiellement de réaliser le broyage et n'est pas adapté pour mettre en oeuvre une analyse plus complète d'un échantillon biologique.

Il existe par ailleurs des solutions qui permettent de réaliser une détection de la présence de pathogène par amplification et détection par colorimétrie ou turbidité ou mesure du pH. De telles solutions sont par exemple décrites dans les publications suivantes :
- « Visual detection of isothermal nucleic acid amplification using pH-sensitive dyes », Nathan A. Tanner et al - BioTechniques, Vol. 58, n°2, February 2015, pp. 59-68.
- « Colorimetric detection of loop-mediated isothermal amplification reaction by using hydroxy naphthol blue », Motoki Goto et al. - BioTechniques, Vol. 46, No. 3, March 2009, pp. 167-172.
- « Loop-Mediated Isothermal Amplification Assay for Rapid Detection of Common Strains of Escherichia coli », Hill J, Beriwal S, Chandra I, et al. - Journal of Clinical Microbiology. 2008;46(8):2800-2804. doi:10.1128/JCM.00152-08.
- « Visual Detection of Norovirus Genogroup II by Reverse Transcription Loop-Mediated Isothermal Amplification with Hydroxynaphthol Blue Dye », Jianming, Ziqian Xu, Kai Nie, Xiong Ding, Li Guan, Ji Wang, Yuying Xian, Xiyang Wu , Xuejun Ma - Food and Environmental Virology, September 2014, Volume 6, Issue 3, pp 196-201.

Il n'existe cependant pas de dispositif qui permette de réaliser une analyse globale et complète d'un échantillon biologique et qui soit :
- Facilement transportable,
- Simple d'utilisation,
- Fiable,
- Peu coûteux.

Le but de l'invention est de proposer un dispositif d'analyse d'un échantillon biologique permettant de remplir ces différents objectifs.

### Exposé de l'invention

Ce but est atteint par un dispositif d'analyse d'un échantillon biologique comprenant des espèces biologiques, ledit dispositif comportant :
- Un boîtier comportant au moins une ouverture,
- Un canal d'injection ménagé dans ledit boîtier et par lequel peut être injecté ledit échantillon biologique sous la forme d'un fluide,
- Un canal d'évacuation ménagé dans ledit boîtier,
- Une chambre dans laquelle débouche le canal d'injection et le canal d'évacuation,
- Un filtre séparant ladite chambre en deux espaces distincts, de manière à définir un premier espace dans lequel débouche ledit canal d'injection et un deuxième espace dans lequel débouche ledit canal d'évacuation, ledit filtre présentant une porosité adaptée à la séparation à effectuer,
- Une surface d'appui rugueuse présentant un paramètre de rugosité de surface adapté pour réaliser une lyse mécanique desdites espèces biologiques, ladite surface d'appui étant agencée dans ledit premier espace, ledit filtre comportant au moins une partie présentant une déformabilité élastique suffisante pour atteindre ladite surface d'appui rugueuse lors de l'exercice d'une force d'appui,
- Une membrane souple agencée à l'opposé de la surface d'appui rugueuse par rapport au filtre et obturant l'ouverture réalisée à travers le boîtier.

Par rapport à l'état de la technique, on comprend notamment que l'architecture du dispositif permet de bien dissocier les fonctions de séparation et de lyse mécanique. Cela présente notamment l'avantage de pouvoir assurer l'une des deux étapes, sans forcément effectuer l'autre. Et cela permet également de modifier les caractéristiques du filtre indépendamment des caractéristiques de la surface d'appui rugueuse pour effectuer la lyse.

En outre l'architecture du dispositif permettra avantageusement de réaliser la séparation dans la chambre, en maintenant dans la chambre à la fois les polluants (dans le premier espace de la chambre) et le matériel biologique à étudier (dans le deuxième espace de la chambre).

Selon une particularité, la membrane est réalisée dans un matériau transparent. Cela permettra notamment de réaliser une détection en visualisant le contenu de la chambre à travers la membrane.

Selon une autre particularité, le boîtier comporte au moins une paroi réalisée dans un matériau transparent.

Selon une autre particularité, le dispositif comporte des moyens de chauffage agencés pour chauffer la chambre à une température déterminée.

Selon une autre particularité, le boîtier comporte une paroi inférieure, une paroi latérale et une paroi supérieure.

Selon une autre particularité, le canal d'injection et le canal d'évacuation sont par exemple réalisés à travers ladite paroi supérieure du boîtier.

Selon une autre particularité, ladite ouverture obturée par la membrane est par exemple réalisée à travers la paroi supérieure du boîtier.

Selon une autre particularité, la surface d'appui rugueuse présente un paramètre de rugosité de surface moyen compris par exemple entre 0.1 µm et 10 µm.

Selon une autre particularité, le filtre présente des pores ayant un diamètre moyen compris par exemple entre 0.2 µm et 0.5 µm.

L'invention concerne également un procédé d'analyse d'un échantillon biologique, mis en oeuvre à l'aide du dispositif tel que décrit ci-dessus, ce procédé comportant ainsi au moins les étapes suivantes :
- Injection d'un échantillon contenant des espèces biologiques dans le premier espace de la chambre du dispositif par le canal d'injection,
- Lyse mécanique des espèces biologiques présentes dans la chambre par broyage contre la surface d'appui rugueuse afin de libérer un matériel biologique à analyser,
- Séparation du matériel biologique par rapport aux polluants par filtrage à travers le filtre en faisant passer le matériel biologique dans le deuxième espace de la chambre et en maintenant des polluants dans le premier espace,
- Amplification du matériel biologique par chauffage de la chambre à une température déterminée,
- Analyse du matériel biologique obtenu après amplification.

Selon une particularité, le procédé peut comporter également une étape de rinçage et de purification des espèces biologiques présentes dans la chambre, mise en oeuvre préalablement à l'étape de lyse mécanique.

Selon une autre particularité, l'étape d'analyse est par exemple mise en oeuvre par colorimétrie, mesure électrochimique, mesure de turbidité ou fluorescence.

### Brève description des figures

D'autres caractéristiques et avantages vont apparaître dans la description détaillée qui suit faite en regard des dessins annexés dans lesquels :

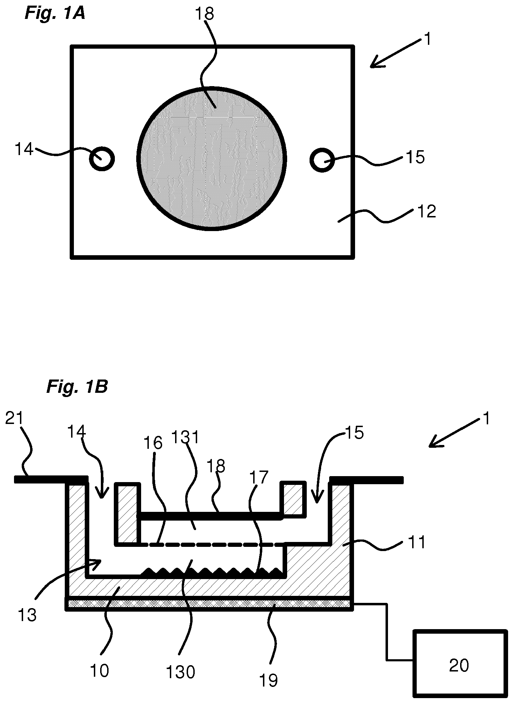

- Les figures 1A et 1B représentent, de manière schématique, respectivement en vue de dessus et en vue de côté, le dispositif d'analyse d'un échantillon biologique selon l'invention.
- Les figures 2A à 2E illustrent les différentes étapes d'une méthode d'analyse et de détection mise en oeuvre grâce au dispositif de l'invention.

### Description détaillée d'au moins un mode de réalisation

Le dispositif de l'invention est destiné à l'analyse d'un échantillon biologique. Cet échantillon biologique se présente par exemple sous la forme d'un fluide qui contient des espèces biologiques contenant un matériel biologique à étudier. Par espèces biologiques, on entend notamment des micro-organismes, des cellules, des spores... Par matériel biologique à étudier, on entend par exemple des molécules d'acide nucléique (ARN, ADN) issues d'une cellule, des protéines, des lipopolysaccharides (LPS), des acides lipotéichoïques (LTA)...

Par fluide, on entend notamment un liquide, un gaz... Le liquide pourra présenter différents degrés de viscosité et pourra par exemple se présenter sous la forme d'une pâte ou d'un gel.

De manière avantageuse, l'invention présente la particularité de pouvoir effectuer, dans un même dispositif, à la fois :
- Une purification et une concentration des espèces biologiques présentes dans l'échantillon biologique,
- Une lyse mécanique des espèces biologiques présentes dans l'échantillon en vue d'en extraire un matériel biologique à étudier,
- Une séparation entre le matériel biologique à étudier et des polluants présents, et
- Une détection de présence de pathogène dans le matériel biologique qui a été séparé.

Dans la suite de la description, les termes "inférieur", "supérieur", "haut" et "bas" employés sont à comprendre en prenant comme référence un axe principal qui est vertical.

Dans la suite de la description, les termes "externe", "extérieur", "interne", "intérieur", doivent être compris en prenant comme référence la chambre du dispositif qui sera décrite ci-dessous.

Le dispositif 1 comporte un boîtier. Le boîtier comporte une paroi inférieure 10, une paroi latérale 11 et une paroi supérieure 12. Toutes les parois du boîtier seront préférentiellement réalisées dans un même matériau. Ce matériau sera notamment apte à pouvoir subir un chauffage dans une fourchette de température comprise entre 20°C et 100°C. Préférentiellement, certaines parois du boîtier, au moins sa paroi latérale 11, seront réalisées dans un matériau transparent. Préférentiellement, le matériau employé sera un plastique, par exemple de type PMMA (Poly(Méthacrylate de Méthyle)).

Le dispositif 1 comporte une chambre 13 ménagée dans le boîtier. Cette chambre représente l'emplacement dans lequel sont effectuées à la fois la purification/concentration, la lyse mécanique, la séparation et éventuellement la détection dans les espèces biologiques. La chambre 13 est fermée vers le bas par la paroi inférieure du boîtier.

Le dispositif comporte un canal d'injection 14 pour y injecter tous types de fluides, par exemple à l'aide d'une pipette. Le canal d'injection comporte une entrée ménagée par exemple à travers la paroi supérieure 12 du boîtier et une sortie qui débouche dans ladite chambre 13. L'entrée du canal d'injection 14 est par exemple agencée verticalement et sa sortie débouche par exemple horizontalement dans la chambre 13. L'entrée du canal d'injection est par exemple évasée pour y appliquer le cône d'une pipette ou sera adaptée au type de dispositif employé pour injecter le fluide dans le dispositif. A titre d'exemple, il s'agira d'une entrée présentant un embout de type lueur pour y connecter une seringue.

Le dispositif comporte un canal d'évacuation 15 dont l'entrée communique avec l'espace formé par la chambre 13 et la sortie communique avec l'extérieur par une ouverture réalisée par exemple à travers la paroi supérieure du boîtier. Par ce canal d'évacuation 15, on évacue des fluides injectés. Son entrée est par exemple agencée horizontalement et sa sortie verticalement. La chambre 13 est placée entre le canal d'injection 14 et le canal d'évacuation 15.

Vers le haut, la chambre 13 est fermée par une membrane 18 souple et étirable, préférentiellement transparente. La paroi supérieure 12 du boîtier du dispositif comporte ainsi une ouverture qui est recouverte de manière hermétique par ladite membrane 18. Ladite membrane est ainsi ancrée dans le boîtier par toute solution de fixation adaptée, par exemple par collage. Cette membrane 18 sera par exemple composée d'un film, par exemple de type MicroAmp, 3M (marques déposées), d'épaisseur, de dimensions et de constitution adaptées pour se déformer de manière élastique, par rapport à ses points d'ancrage, notamment jusqu'au fond de la chambre 13.

Par le terme "transparent", on entend que le matériau employé est au moins partiellement transparent à la lumière visible, de manière à laisser passer au moins 80% de cette lumière. Il faut ainsi comprendre qu'il sera suffisamment transparent pour voir l'intérieur de la chambre 13, au moins le deuxième espace situé au-dessus du filtre.

Le dispositif comporte un filtre 16 agencé dans ladite chambre 13 et séparant ladite chambre 13 en deux espaces. Les deux espaces sont par exemple superposés et désignés ainsi espace inférieur 130 situé sous le filtre et espace supérieur 131 situé au-dessus du filtre. Ce filtre 16 est préférentiellement réalisé en tout ou partie sous la forme d'un film souple et fin, tiré dans l'espace formé par la chambre de manière à ne permettre le passage d'un espace à l'autre que par les pores du filtre 16. Le film présente une déformabilité élastique lui permettant de s'étirer lors de l'exercice d'une force d'appui dans une direction sensiblement verticale, cette déformabilité élastique ayant un niveau suffisant pour atteindre la surface inférieure de la chambre 13. Le filtre 16 présente un diamètre moyen de pores compris entre 0.2 µm et 50 µm, par exemple compris entre 0.2 µm et 1 µm pour la séparation de microorganismes. Le diamètre des pores est bien entendu adapté pour assurer une séparation entre les polluants et le matériel biologique à étudier. Après l'étape de lyse et la séparation par le filtre 16, le matériel biologique à étudier reste au-dessus du filtre 16, dans l'espace supérieur 131 de la chambre, tandis que les polluants restent au-dessous du filtre, dans l'espace inférieur 130 de la chambre. Le filtre 16 sera par exemple composé d'un film d'épaisseur, de dimensions et de constitution adaptée pour se déformer jusqu'au fond de la chambre 13 par rapport à ses points d'ancrage. Selon un mode de réalisation particulier, le filtre pourra être aussi réalisé dans un matériau transparent, par exemple avec les mêmes caractéristiques de transparence que la membrane.

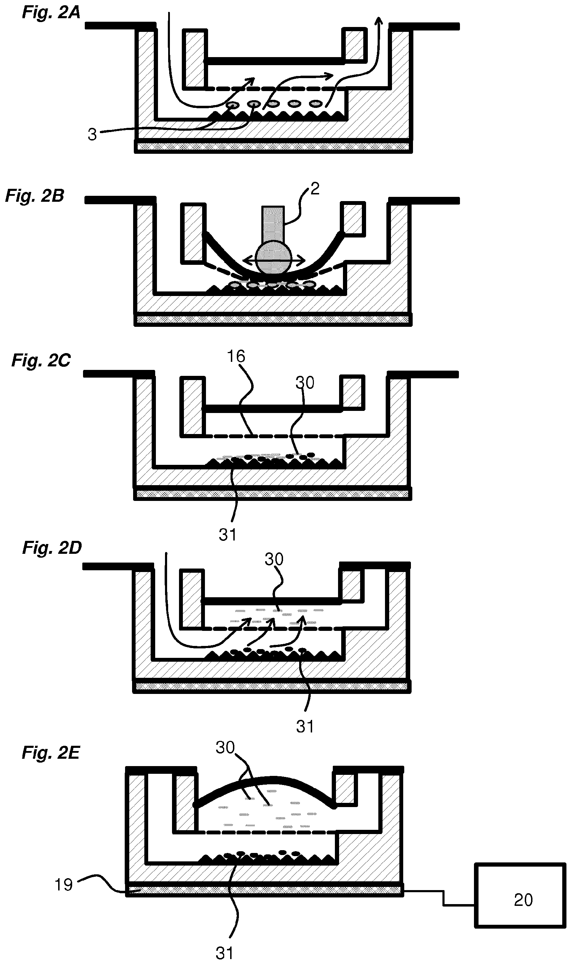

Le dispositif comporte une surface d'appui rugueuse 17 agencée sur le fond de la chambre 13. Cette surface d'appui rugueuse 17 s'étend sur une partie majoritaire du fond de la chambre. Elle comporte un paramètre de rugosité de surface moyen compris entre 0.1µm et 10 µm, préférentiellement compris entre 0.2 µm et 3 µm. Cette surface d'appui rugueuse 17 est destinée à permettre une lyse mécanique des espèces biologiques présentes dans un échantillon biologique placé dans le dispositif. Préférentiellement, la lyse mécanique est réalisée en broyant lesdites espèces biologiques, par abrasion sur ladite surface d'appui rugueuse. L'opération de broyage est mise en oeuvre par un mouvement de friction des espèces biologiques contre la surface d'appui rugueuse, en employant un organe de de broyage adapté. Cet organe sera par exemple une spatule 2 (voir figure 2B) ou une tige, par exemple en matériau plastique ou métallique. Cet organe est appliqué de l'extérieur de la chambre 13 et son extrémité est appliquée contre la surface externe de la membrane 18 de manière à étirer la membrane 18 et le filtre vers le fond de la chambre et ainsi frictionner les espèces biologiques présentes dans un échantillon contre la surface d'appui rugueuse.

Le dispositif comportera préférentiellement des moyens pour obturer le canal d'injection et le canal d'évacuation afin de fermer tout accès à la chambre et d'isoler l'espace interne de la chambre par rapport à l'extérieur. Ces moyens sont par exemple formés de deux volets 21 dont la position permet d'obturer ou d'ouvrir chaque canal 14, 15 ou formés d'adhésifs collés sur l'entrée du canal d'injection et la sortie du canal d'évacuation. D'autres solutions pourraient bien entendu être envisagées.

Préférentiellement, le boîtier peut intégrer des moyens de chauffage de l'espace interne de la chambre, composés par exemple d'au moins une résistance 19 chauffante, comme représenté sur les figures annexées. La résistance est par exemple fixée sous la paroi inférieure du boîtier. Une source d'alimentation 20 sera par exemple prévue pour alimenter la résistance 19. La source d'alimentation comportera par exemple une ou plusieurs piles électriques, fournissant suffisamment d'énergie pour chauffer la chambre à une température comprise dans la fourchette définie ci-dessus, c'est-à-dire de 20°C à 100°C. Bien entendu, d'autres moyens de chauffage pourraient être employés, comprenant par exemple une encre conductrice déposée par imprimerie ou sérigraphie sous la paroi inférieure du boîtier.

Ainsi, pour résumer, le dispositif comporte la structure « multicouches » suivante :
- Une surface inférieure d'appui rugueuse 17,
- Un espace inférieur 130 d'une chambre 13 situé au-dessus de la surface d'appui rugueuse 17,
- Un filtre 16 souple et étirable situé au-dessus de l'espace inférieur 130,
- Un espace supérieur 131 de la chambre 13 situé au-dessus du filtre 16,
- Une membrane 18 souple et étirable située au-dessus de l'espace supérieur 131, fermant hermétiquement la chambre et accessible depuis l'extérieur du dispositif.

A l'aide du dispositif décrit ci-dessus, de manière non limitative, un procédé de lyse, de séparation et de détection comportera par exemple les étapes suivantes :
- L'échantillon biologique liquide, comprenant des espèces biologiques 3, est injecté dans la chambre 13 du dispositif 1 à l'aide d'une pipette, via le canal d'injection 14. A titre d'exemple, l'échantillon présente un volume de 1 millilitre et contient 10⁵ bactéries par millilitre. La partie liquide de l'échantillon et toutes les particules/molécules qui passent à travers le filtre 16 sont récupérées par le canal d'évacuation 16 et écartées de l'analyse. Les bactéries sont alors concentrées dans l'espace inférieur 130 de la chambre 13. Des polluants présents dans l'échantillon, trop gros pour traverser le filtre, peuvent également rester dans l'espace inférieur 130 de la chambre 13.

- Une fois l'échantillon présent dans la chambre, une solution de lavage/rinçage des espèces biologiques 3 présentes est préférentiellement injectée à l'aide d'une pipette via le canal d'injection 14, de manière à purifier les bactéries présentes dans l'échantillon (figure 2A. Des polluants 31, trop gros pour traverser le filtre 16 et non solubles, peuvent également rester dans l'espace 130.
- Une lyse mécanique des espèces biologiques 3 est mise en oeuvre à l'aide d'une spatule 2. L'extrémité de la spatule 2 est mise au contact de la surface externe de la membrane 18, puis en appuyant sur la membrane 18 pour l'étirer vers l'intérieur de la chambre 13, l'extrémité de la spatule 2 atteint la surface d'appui rugueuse 17 de manière à broyer les espèces biologiques 3 contre la surface d'appui rugueuse (figure 2B).
- Une fois la lyse mécanique effectuée, l'espace inférieur de la chambre comporte le matériel biologique 30 à étudier, par exemple des molécules d'ADN, et des polluants 31 (figure 2C).
- Pour effectuer la séparation entre le matériel biologique 30 à étudier et les polluants 31, une solution liquide contenant des réactifs d'amplification est injectée dans la chambre par le canal d'injection 14, pour éluer le matériel biologique à étudier. Une partie de la solution liquide injectée emporte ainsi le matériel biologique 30 à étudier, par exemple les molécules d'ADN, passe à travers le filtre 16 et rejoint l'espace supérieur 131 de la chambre. Les polluants 31, qui présentent une structure supérieure au diamètre des pores du filtre 16, restent dans l'espace inférieur 130 de la chambre 13. Lors de cette étape, le canal d'évacuation 15 est préférentiellement obturé par un volet 21 de manière à éviter toute évacuation du matériel biologique 30 à étudier (figure 2D).
- Une fois que la séparation entre les polluants 31 et le matériel biologique 30 à étudier est effectuée, il s'agit de réaliser une réaction d'amplification du matériel biologique afin, par exemple, de pouvoir détecter la présence d'un pathogène dans le matériel biologique 30 qui a été séparé.
- La réaction d'amplification est mise en oeuvre en chauffant l'espace interne de la chambre 13, par exemple à l'aide du système de résistances 19 intégré au boîtier. Lors de l'opération de chauffage, le canal d'injection 14 et le canal d'évacuation 15 sont préférentiellement fermés. La température à laquelle est chauffée la chambre 13 dépend du type de réaction d'amplification mise en oeuvre. Il pourra s'agir de tous types de réaction d'amplification, par exemple LAMP ("Loop-Mediated Isothermal Amplification"), PCR ("Polymerase Chain Reaction"), NASBA ("Nucleic Acid Sequence Based Amplification"), RPA ("Recombinase Polymerase Amplification") ... Pour une amplification de type LAMP, le chauffage est réalisé à une température avantageusement comprise entre 60°C et 65°C. Cette réaction permet d'amplifier les molécules du matériel biologique 30 à détecter, par exemple les molécules d'ADN. Lors du chauffage, la membrane 18 est par exemple amenée à s'étirer pour créer une concavité dans la chambre 13, augmentant ainsi le volume de la chambre (figure 2E).
- Lors de la réaction d'amplification du matériel biologique, il s'agit de détecter si un pathogène est présent. Pour cela, il est possible d'employer différentes méthodes, comme par exemple la colorimétrie, la fluorescence, l'électrochimie, la pHmétrie, la mesure de turbidité. Toute autre méthode de détection pourrait être envisagée. Pour une méthode de détection de type PHmétrie, des électrodes de détection de pH pourraient être intégrées au dispositif. Lors d'une détection par colorimétrie ou fluorescence, la caractéristique transparente de la membrane 18 et/ou du boîtier du dispositif, notamment au niveau de sa paroi latérale, présente l'avantage de permettre une détection visuelle directe. Ainsi, en observant la couleur de l'échantillon présent dans la chambre, il est possible de déterminer si un pathogène est présent ou non.

Le dispositif de l'invention comporte ainsi de nombreux avantages, parmi lesquels :
- Un dispositif facilement transportable, car léger et peu encombrant.
- Un dispositif qui permet de faire dans une même chambre, à la fois la concentration/purification, la lyse, la séparation, l'amplification et la détection, formant ainsi un laboratoire sur puce (« Lab On Chip »).
- Un dispositif qui ne comporte aucune pièce mécanique complexe pour réaliser la lyse mécanique.
- Un dispositif qui permet éventuellement une détection par colorimétrie, juste en regardant à travers les parties transparentes du dispositif.

## Revendications

1. Dispositif d'analyse d'un échantillon biologique comprenant des espèces biologiques (3), ledit dispositif comportant :
- Un boîtier comportant au moins une ouverture,
- Un canal d'injection (14) ménagé dans ledit boîtier et par lequel peut être injecté ledit échantillon biologique sous la forme d'un fluide,
- Un canal d'évacuation (15) ménagé dans ledit boîtier,
- Une chambre (13) dans laquelle débouche le canal d'injection (14) et le canal d'évacuation (15),
- Un filtre (16) séparant ladite chambre en deux espaces distincts de manière à définir un premier espace (130) dans lequel débouche ledit canal d'injection (14) et un deuxième espace (131) dans lequel débouche ledit canal d'évacuation (15), ledit filtre (16) présentant une porosité adaptée à la séparation à effectuer,
- Une surface d'appui rugueuse (17) présentant un paramètre de rugosité de surface adapté pour réaliser une lyse mécanique desdites espèces biologiques (3), ladite surface d'appui étant agencée dans ledit premier espace (130) et ledit filtre comportant au moins une partie présentant une déformabilité élastique suffisante pour atteindre ladite surface d'appui rugueuse de la chambre (13) lors de l'exercice d'une force d'appui,
- Une membrane (18) souple agencée à l'opposé de la surface d'appui rugueuse (17) par rapport au filtre (16) et obturant l'ouverture réalisée à travers le boîtier.

2. Dispositif selon la revendication 1, **caractérisé en ce que** la membrane (18) est réalisée dans un matériau transparent.

3. Dispositif selon la revendication 1 ou 2, **caractérisé en ce que** le boîtier comporte au moins une paroi réalisée dans un matériau transparent.

4. Dispositif selon l'une des revendications 1 à 3, **caractérisé en ce qu'**il comporte des moyens de chauffage agencés pour chauffer la chambre (13) à une température déterminée.

5. Dispositif selon l'une des revendications 1 à 4, **caractérisé en ce que** le boîtier comporte une paroi inférieure (10), une paroi latérale (11) et une paroi supérieure (12).

6. Dispositif selon la revendication 5, **caractérisé en ce que** le canal d'injection (14) et le canal d'évacuation sont réalisés à travers ladite paroi supérieure du boîtier.

7. Dispositif selon la revendication 5 ou 6, **caractérisé en ce que** ladite ouverture obturée par la membrane (18) est réalisée à travers la paroi supérieure (12) du boîtier.

8. Dispositif selon l'une des revendications 1 à 7, **caractérisé en ce que** la surface d'appui rugueuse présente un paramètre de rugosité de surface moyen compris entre 0.1µm et 10 µm.

9. Dispositif selon l'une des revendications 1 à 8, **caractérisé en ce que** le filtre présente des pores ayant un diamètre moyen compris entre 0.2 µm et 0.5 µm.

10. Procédé d'analyse d'un échantillon biologique, mis en oeuvre à l'aide du dispositif défini dans l'une des revendications 1 à 9, **caractérisé en ce qu'**il comporte les étapes suivantes :
- Injection d'un échantillon contenant des espèces biologiques (3) dans le premier espace (130) de la chambre (13) du dispositif par le canal d'injection (14),
- Lyse mécanique des espèces biologiques présentes dans la chambre par broyage contre la surface d'appui rugueuse (17) afin de libérer un matériel biologique (30) à analyser,
- Séparation du matériel biologique (30) par rapport aux polluants (31) par filtrage à travers le filtre (16) en faisant passer le matériel biologique (30) dans le deuxième espace (131) de la chambre et en maintenant des polluants dans le premier espace,
- Amplification du matériel biologique par chauffage de la chambre à une température déterminée,
- Analyse du matériel biologique obtenu après amplification.

11. Procédé selon la revendication 10, **caractérisé en ce qu'**il comporte une étape de rinçage et de purification des espèces biologiques présentes dans la chambre (13), mise en oeuvre préalablement à l'étape de lyse mécanique.

12. Procédé selon la revendication 10 ou 11, **caractérisé en ce que** l'étape d'analyse est mise en oeuvre par colorimétrie, mesure électrochimique, mesure de turbidité ou fluorescence.

## Patentansprüche

1. Analysevorrichtung für eine biologische Probe, die biologische Spezies (3) umfasst, wobei die Vorrichtung aufweist:
- ein Gehäuse, das mindestens eine Öffnung aufweist,
- einen Einspritzkanal (14), der in dem Gehäuse ausgebildet ist und durch den die biologische Probe in Form eines Fluids eingespritzt werden kann,
- einen Auslasskanal (15), der in dem Gehäuse ausgebildet ist,
- eine Kammer (13), in die der Einspritzkanal (14) und der Auslasskanal (15) mündet,
- einen Filter (16), der die Kammer in zwei getrennte Räume derart trennt, um einen ersten Raum (130), in den der Einspritzkanal (14) mündet, und einen zweiten Raum (131), in den der Auslasskanal (15) mündet, zu definieren, wobei der Filter (16) eine Porosität aufweist, die für das durchzuführende Trennen geeignet ist,
- eine raue Auflagefläche (17), die einen Oberflächenrauhigkeitsparameter aufweist, der geeignet ist, um eine mechanische Lyse der biologischen Spezies (3) durchzuführen, wobei die Auflagefläche in dem ersten Raum (130) ausgebildet ist und wobei der Filter mindestens einen Teil aufweist, der eine elastische Verformbarkeit aufweist, die ausreichend ist, um die raue Auflagefläche der Kammer (13) beim Ausüben von einer Anpresskraft zu erreichen,
- eine flexible Membran (18), die gegenüberliegend von der rauen Auflagefläche (17) in Bezug auf den Filter (16) angeordnet ist und die Öffnung verschließt, die durch das Gehäuse hindurch hergestellt ist.

2. Vorrichtung nach Anspruch 1, **dadurch gekennzeichnet, dass** die Membran (18) aus einem durchsichtigem Material hergestellt ist.

3. Vorrichtung nach Anspruch 1 oder 2, **dadurch gekennzeichnet, dass** das Gehäuse mindestens eine Wand aufweist, die aus einem durchsichtigem Material hergestellt ist.

4. Vorrichtung nach einem der Ansprüche 1 bis 3, **dadurch gekennzeichnet, dass** sie Heizmittel aufweist, die angeordnet sind, um die Kammer (13) auf eine bestimmte Temperatur zu erhitzen.

5. Vorrichtung nach einem der Ansprüche 1 bis 4, **dadurch gekennzeichnet, dass** das Gehäuse eine untere Wand (10), eine seitliche Wand (11) und eine obere Wand (12) aufweist.

6. Vorrichtung nach Anspruch 5, **dadurch gekennzeichnet, dass** der Einspritzkanal (14) und der Auslasskanal durch die obere Wand des Gehäuses hindurch hergestellt sind.

7. Vorrichtung nach Anspruch 5 oder 6, **dadurch gekennzeichnet, dass** die Öffnung, die von der Membran (18) verschlossen ist, durch die obere Wand (12) des Gehäuses hindurch hergestellt ist.

8. Vorrichtung nach einem der Ansprüche 1 bis 7, **dadurch gekennzeichnet, dass** die raue Auflagefläche einen mittleren Oberflächenrauhigkeitsparameter aufweist, der zwischen 0,1 µm und 10 µm beträgt.

9. Vorrichtung nach einem der Ansprüche 1 bis 8, **dadurch gekennzeichnet, dass** der Filter Poren aufweist, die einen mittleren Durchmesser aufweisen, der zwischen 0,2 µm und 0,5 µm beträgt.

10. Analyseverfahren einer biologischen Probe, das mit Hilfe der Vorrichtung durchgeführt wird, die in einem der Ansprüche 1 bis 9 definiert ist, **dadurch gekennzeichnet, dass** es die folgenden Schritte aufweist:
- Einspritzen einer Probe, die biologische Spezies (3) umfasst, in den ersten Raum (130) der Kammer (13) der Vorrichtung durch den Einspritzkanal (14),
- mechanische Lyse der biologischen Spezies, die in der Kammer vorhanden sind, durch Zerkleinern gegen die raue Auflagefläche (17) um ein zu analysierendes biologisches Material (30) freizusetzen,
- Trennen des biologischen Materials (30) in Bezug auf die Verunreinigungen (31) durch Filtern durch den Filter (16), in dem das biologische Material (30) in den zweiten Raum (131) der Kammer geführt wird und indem Verunreinigungen in dem ersten Raum gehalten werden,
- Amplifikation des biologischen Materials durch Heizen der Kammer auf eine bestimmte Temperatur,
- Analyse des biologischen Materials, das nach der Amplifikation erhalten wird.

11. Verfahren nach dem Anspruch 10, **dadurch gekennzeichnet, dass** es einen Schritt des Spülens und des Reinigens der biologischen Spezies aufweist, die in der Kammer (13) enthalten sind, der vor dem Schritt der mechanischen Lyse durchgeführt wird.

12. Verfahren nach Anspruch 10 oder 11, **dadurch gekennzeichnet, dass** der Schritt der Analyse durch Kolorimetrie, elektrochemische Messung, Messen von Trübheit oder Fluoreszenz durchgeführt wird.

## Claims

1. Device for analyzing a biological sample comprising biological species (3), said device comprising:
- A housing comprising at least one opening,
- An injection channel (14) made in said housing and via which said biological sample can be injected in the form of a fluid,
- A discharge channel (15) made in said housing,
- A chamber (13) into which the injection channel (14) and the discharge channel (15) open,
- A filter (16) separating said chamber into two distinct spaces so as to define a first space (130) into which said injection channel (14) opens and a second space (131) into which said discharge channel (15) opens, said filter (16) having a porosity suitable for the separation to be carried out,
- A rough bearing surface (17) having a surface roughness parameter suitable for carrying out a mechanical lysis of said biological species (3), said bearing surface being arranged in said first space (130) and said filter comprising at least one part having an elastic deformability sufficient to reach said rough bearing surface of the chamber (13) during the exertion of a bearing force,
- A flexible membrane (18) arranged opposite the rough bearing surface (17) relative to the filter (16) and blocking the opening made through the housing.

2. Device according to Claim 1, **characterized in that** the membrane (18) is made of a transparent material.

3. Device according to Claim 1 or 2, **characterized in that** the housing comprises at least one wall made of a transparent material.

4. Device according to one of Claims 1 to 3, **characterized in that** it comprises heating means arranged so as to heat the chamber (13) to a given temperature.

5. Device according to one of Claims 1 to 4, **characterized in that** the housing comprises a lower wall (10), a side wall (11) and an upper wall (12).

6. Device according to Claim 5, **characterized in that** the injection channel (14) and the discharge channel are made through said upper wall of the housing.

7. Device according to Claim 5 or 6, **characterized in that** said opening blocked by the membrane (18) is made through the upper wall (12) of the housing.

8. Device according to one of Claims 1 to 7, **characterized in that** the rough bearing surface has a mean surface roughness parameter of between 0.1 µm and 10 µm.

9. Device according to one of Claims 1 to 8, **characterized in that** the filter has pores which have a mean diameter of between 0.2 µm and 0.5 µm.

10. Method for analyzing a biological sample, carried out by means of the device defined in one of Claims 1 to 9, **characterized in that** it comprises the following steps:
- Injection of a sample containing biological species (3) into the first space (130) of the chamber (13) of the device by the injection channel (14),
- Mechanical lysis of the biological species present in the chamber by grinding against the rough bearing surface (17) in order to release a biological material (30) to be analyzed,
- Separation of the biological material (30) with respect to the pollutants (31) by filtering through the filter (16) while passing the biological material (30) into the second space (131) of the chamber and while maintaining pollutants in the first space,
- Amplification of the biological material by heating the chamber to a given temperature,
- Analysis of the biological material obtained after amplification.

11. Method according to Claim 10, **characterized in that** it comprises a step of rinsing and of purification of the biological species present in the chamber (13), carried out prior to the mechanical lysis step.

12. Method according to Claim 10 or 11, **characterized in that** the analysis step is carried out by colorimetry, electrochemical measurement, turbidity measurement or fluorescence.
